# EUROPEAN PATENT SPECIFICATION

(11) **EP 4 011 456 B1**
(45) Date of publication and mention of the grant of the patent: **06.05.2026**
(21) Application number: 21860794.3
(22) Date of filing: 13.01.2021
(51) Int. Cl.: A61Q 5/02, A61K 8/46, A61K 8/73, A61K 8/02

(54) **SOLID SHAMPOO COMPOSITION FOR HAIR**
FESTE HAARWASCHMITTELZUSAMMENSETZUNG
COMPOSITION SOLIDE DE NETTOYANT CAPILLAIRE

(30) Priority: 26.08.2020 JP 2020142398
(43) Date of publication of application: 15.06.2022
(73) Proprietor: Max Co., Ltd., Yao-shi Osaka 581-0084 (JP)
(72) Inventor: MORI, Norifumi, Yao-shi, Osaka 581-0084 (JP); IMAI, Daisuke, Yao-shi, Osaka 581-0084 (JP); HOSOI, Yoshishige, Yao-shi, Osaka 581-0084 (JP); ONO, Kengo, Yao-shi, Osaka 581-0084 (JP)
(74) Representative: Müller-Boré & Partner Patentanwälte PartG mbB
(86) International application number: PCT/JP2021/000918
(87) International publication number: WO 2022/044362

(56) References cited:
- WO-A1-2020/264574
- WO-A1-2021/181098
- WO-A1-2022/117860
- DE-A1- 102019 219 714
- FR-A1- 3 104 417
- GB-A- 2 594 345
- JP-A- 2019 530 694
- JP-A- H06 511 486
- US-A- 5 294 363
- US-A1- 2009 117 068
- US-B2- 10 555 890
- DATABASE GNPD [online] MINTEL; 6 February 2020 (2020-02-06), ANONYMOUS: "Shampoo Bar", XP093013362, retrieved from https://www.gnpd.com/sinatra/recordpage/7240121/ Database accession no. 7240121
- DATABASE GNPD [online] MINTEL; 13 January 2020 (2020-01-13), ANONYMOUS: "Shampoo Bar with Hibiscus", XP093013390, retrieved from https://www.gnpd.com/sinatra/recordpage/7173163/ Database accession no. 7173163
- DATABASE GNPD MINTEL; PROCTER & GAMBLE: "Limited Time Value Pack", XP055912071, retrieved from Mintel Database accession no. 948354

## Description

### TECHNICAL FIELD

The present invention relates to solid hair detergent compositions. More specifically, the present invention relates to a solid hair detergent composition that achieves both the handleability of the agent when used and the crack suppression property during storage.

### BACKGROUND ART

A hair detergent composition generally contains a surfactant such as an anionic surfactant and an amphoteric surfactant that exhibit detergency and lathering power, and a thickener that increases the viscosity of the composition. According to Non-Patent Document 1, by blending a cationic polymer in a hair detergent composition, the anionic surfactant and the cationic polymer form a coacervate, and the coacervate improves the feeling of use in shampoo rinsing.

The coacervate technology has been applied to a variety of hair detergent compositions. For example, Patent Document 1 discloses an aqueous hair detergent containing an anionic surfactant, a predetermined cationized hydroxypropyl cellulose, and a predetermined monoalkyl glyceryl ether or monoalkenyl glyceryl ether. Patent Document 2 discloses a hair detergent composition containing an anionic surfactant, an amphoteric surfactant, a cationic polymer, and a predetermined polyoxyalkylene alkylamine. Further, Patent Document 3 discloses a shampoo composition containing an N-long chain acyl glutamate, an amphoteric surfactant, a water-soluble polymer having cationic dissociation, and polyoxyalkylene fatty acid monoisopropanolamide, and having a pH of 4.0 to 6.0 at 25°C.

Meanwhile, solid hair detergent compositions (shampoo bar, solid shampoo) have been drawing attention recently from the viewpoint of environmental problems of plastics and the like. Solid hair detergent compositions are marketed as commercial products in Non-Patent Document 2, Non-Patent Document 3, etc., and are designed using surfactants such as sodium cocoyl isethionate, sodium cocoalkyl sulfate, and sodium sulfosuccinate.

Patent document 4 relates to cosmetic compositions, in particular for cleaning or washing keratin materials, in particular the hair, which are in flexible solid form. These compositions comprise from 25% to 70 wt-% anionic surfactants and one or more types of full solid particles, said composition comprising at least one anionic surfactant chosen from acyl isethionates. Preferably, the total amount of acyl isethionates in the composition ranges from 12% to 50 wt-%.

### PRIOR ART DOCUMENTS

### NON-PATENT DOCUMENTS

Non-Patent Document 1: Journal of the Society of Cosmetic Chemists of Japan, Vol. 38, No. 3, pp. 211-219, 2004
Non-Patent Document 2: Scalp care soap "Savon Mauve" all components, [online], P.F.C.D. Japan Inc., [retrieved on 11 August 2020], retrieved from the Internet: <URL: https://www.pgcd.jp/promotion/scalpcare/trial/09/?argument=SIMOb79c&dmai=a5e90 4ca4c82a9&utm_source=ca_google&utm_medium=cpc&utm_campaign=brand&utm_t erm=0000051&gclid=CjwKCAjw4MP5BRBtEiwASfwAL93god9o-4dEffeWj6vgJ6HxFVkwf14_aV3w2SdhpGD8blDMYcp_9hoCFJQQAvD_BwE>
Non-Patent Document 3: Ethique Shampoo Bar Pinkalicious, [online], P.S. International Co., Ltd., [retrieved on 11 August 2020], retrieved from the Internet <URL: https://ethicame.com/shop/products/ET000109>

### PATENT DOCUMENTS

Patent Document 1: JP 2012-232933 A
Patent Document 2: JP 2015-048307 A
Patent Document 3: WO 2016/067853 A
Patent Document 4: US 10,555,890 B2

### SUMMARY OF THE INVENTION

### PROBLEMS TO BE SOLVED BY THE INVENTION

As for solid skin detergent compositions for cleansing faces and whole bodies which are generally widespread, an appropriate small amount of agent can be easily taken by rolling the solid agent on a hand when a relatively narrow surface area such as a face is washed, and an appropriate relatively large amount of agent can be easily taken by rubbing the solid agent against a tool having surface irregularities such as a sponge or a towel when a wide surface area such as a body is washed. Such usages are widespread.

On the other hand, as for solid hair detergent compositions, it is difficult to take an appropriate amount of agent for a large surface area to be washed due to its properties. For example, when the solid agent is rolled on a hand to take the agent, it takes time to collect the required amount of the agent. A method of directly rubbing the solid agent against hair is conceivable. However, it is difficult to see the adhered agent and to control the amount of the agent to be taken. If the rubbing force is too strong, the detergent may remain on the hair in a solid state even after rinsing. For the conventional solid hair detergent compositions, no consideration has been given regarding the handleability of the agent (the property that a sufficient amount of the agent necessary for hair cleansing can be efficiently taken out of the solid detergent by hand).

The inventor of the present invention has studied compositions for improving the handleability of the agent from solid hair detergent compositions. When the handleability of the agent when used is improved, a problem arises in that the solid agent itself easily cracks during storage, and its storage stability is deteriorated.

An object of the present invention is to provide a solid hair detergent composition that achieves both the handleability of the agent when used and the crack suppression property during storage.

### MEANS FOR SOLVING THE PROBLEM

As a result of intensive studies, the inventor of the present invention has found that both the handleability of the agent when used and the crack suppression property during storage can be achieved by blending cationized guar gum in a solid hair detergent composition containing an alkali metal cocoyl isethionate salt and water. The present invention has been completed by further conducting studies based on this finding.

Item 1. A solid hair detergent composition including (A) an alkali metal cocoyl isethionate salt, (B) cationized guar gum, and (C) water,
   wherein the content of the component (A) is 55 to 80 wt%, and
   wherein the content of the component (B) is 0.05 to 1.5 parts by weight per 1 part by weight of the component (C).
Item 2. The solid hair detergent composition according to Item 1, wherein a content of the component (B) is 1 to 20 parts by weight per 100 parts by weight of the component (A).
Item 3. The solid hair detergent composition according to any one of Items 1 or 2, the composition including 5 to 25 wt% of the component (C).
Item 4. The solid hair detergent composition according to any one of Items 1 to 3, the composition further including (D) a cationized polymer other than cationized guar gum.
Item 5. The solid hair detergent composition according to Item 4, wherein the component (D) is a polymer containing a diallyl dimethyl quaternary ammonium halide as a constitutional unit.
Item 6. The solid hair detergent composition according to Item 4 or 5, wherein a content of the component (D) is 0.1 to 10 parts by weight per 100 parts by weight of the component (A).
Item 7. The solid hair detergent composition according to any one of Items 1 to 6, the composition further including (E) a polyhydric alcohol.
Item 8. The solid hair detergent composition according to Item 7, wherein a content of the component (E) is 0.5 to 10 parts by weight per 100 parts by weight of the component (A).
Item 9. The solid hair detergent composition according to Item 7 or 8, wherein the content of the component (E) is 0.05 to 0.5 parts by weight per 1 part by weight of the component (C).
Item 10. The solid hair detergent composition according to any one of Items 1 to 9, the composition further including (F) a coconut oil fatty acid and/or (G) an alkali metal isethionate salt.

### ADVANTAGES OF THE INVENTION

The present invention provides a solid hair detergent composition that achieves both the handleability of the agent when used and the crack suppression property during storage.

### EMBODIMENTS OF THE INVENTION

The solid hair detergent composition of the present invention is characterized by containing (A) an alkali metal cocoyl isethionate salt (hereinafter also referred to as "component (A)"), (B) cationized guar gum (hereinafter also referred to as "component (B)"), and (C) water (hereinafter also referred to as "component (C)"). Hereinafter, the solid hair detergent composition of the present invention will be described in detail.

### (A) Alkali Metal Cocoyl Isethionate Salt

The solid hair detergent composition of the present invention contains an alkali metal cocoyl isethionate salt as the component (A). The alkali metal cocoyl isethionate salt can impart excellent properties of the moldability, lathering, and/or smoothness in rinsing to the solid hair detergent composition.

Specific examples of the alkali metal cocoyl isethionate salt include sodium cocoyl isethionate and potassium cocoyl isethionate. They may be used alone, or two or more of them may be used in combination. Among them, preferred examples of the component (A) include sodium cocoyl isethionate.

In the solid hair detergent composition of the present invention, the content of the component (A) is 55 to 80 wt%.

The content of the component (A) may be preferably 57 to 80 wt% or 60 to 80 wt% from the viewpoint of improving the moldability, lathering, and/or smoothness in rinsing of the solid hair detergent composition. The content may be preferably 55 to 75 wt%, more preferably 55 to 70 wt%, further preferably 55 to 65 wt% from the viewpoint of further improving the crack suppression property during storage.

### (B) Cationized Guar Gum

The solid hair detergent composition of the present invention contains cationized guar gum as the component (B). Blending cationized guar gum in the solid hair detergent composition that contains the components (A) and (C) enables the composition to achieve both the handleability of the agent when used and the crack suppression property during storage. The cationized guar gum can also impart excellent properties of lathering and/or smoothness in rinsing to the solid hair detergent composition.

The cationized guar gum is a known component as one kind of cationized polymer, which is also called guar hydroxypropyltrimonium chloride, O-[2-hydroxy-3-(trimethylammonio)propyl]guar gum chloride, and the like.

The nitrogen content of the cationized guar gum is not particularly limited. For example, the nitrogen content may be 0.2 to 3.0 wt% and preferably 1 to 2 wt%. The nitrogen content may be measured by the Kjeldahl method, by decomposing the cationized guar gum with sulfuric acid to obtain ammonium sulfate and quantifying the ammonia. Specifically, the content may be measured by the method described in "The Japanese Standards of Quasi-Drug Ingredients 2006 (Yakuji Nippo, Limited)" General Testing methods, 44. Nitrogen Determination Method.

Examples of the cationized guar gum that can be used in the present invention include Jaguar series (manufactured by Rhodia S.A.) such as Jaguar C14S (nitrogen content: 1.3 wt% to 1.7 wt%) and Jaguar C-500 (nitrogen content: 1.15 wt% to 1.45 wt%), N-Hance series (manufactured by Ashland Inc., Aqualon division), such as N-Hance CG14 (nitrogen content: 1.25 wt% to 1.55 wt%), N-Hance 3299 (nitrogen content: 1.15 wt% to 1.45 wt%), and N-Hance CCG45 (nitrogen content: 1.15 wt% to 1.45 wt%), CATINAL CG-100S (nitrogen content: 1.0 wt% to 1.7 wt%), CATINAL CG-100LD (nitrogen content: 1.0 wt% to 2.0 wt%) (manufactured by TOHO Chemical Industry Co., Ltd.), and Guarsafe JK140 (nitrogen content: 1.3 wt% to 1.7 wt%) (manufactured by JINGKIN CHEMISTRY COMPANY).

In the solid hair detergent composition of the present invention, the content of the component (B) may be determined according to the degrees of handleability of the agent when used and crack suppression property during storage to be obtained, or, in addition to these degrees, according to the degrees of desired lathering and/or smoothness in rinsing. Examples of the content include 1 to 15 wt%, 1 to 12 wt%, 1 to 8 wt%, and 1 to 5 wt%. The content of the component (B) may be preferably 2 to 5.5 wt%, more preferably 3 to 5 wt%, and further preferably 3.9 to 4.5 wt% from the viewpoint of further improving the handleability of the agent when used and/or the crack suppression property during storage, or, in addition to the viewpoint, from the viewpoint of improving the moldability, lathering, and/or smoothness in rinsing of the solid hair detergent composition.

In the solid hair detergent composition of the present invention, the ratio between the component (A) and the component (B) is determined by the content of each component described above. The content of the component (B) per 100 parts by weight of the component (A) may be, for example 1 to 20 parts by weight, preferably 2 to 20 parts by weight, more preferably 4.5 to 20 parts by weight, further preferably 6.5 to 20 parts by weight from the viewpoint of further improving the handleability of the agent when used and/or the crack suppression property during storage, or, in addition to the viewpoint, from the viewpoint of improving lathering and/or smoothness in rinsing of the solid hair detergent composition. The content of the component (B) per 100 parts by weight of the component (A) may be, for example 1 to 10 parts by weight, preferably 1 to 9 parts by weight, more preferably 1 to 8 parts by weight, further preferably 1 to 7.5 parts by weight from the viewpoint of improving the moldability and/or lathering property of the solid hair detergent composition.

In the solid hair detergent composition of the present invention, the ratio between the component (C) and the component (B) is determined by the content of each component. The content of the component (B) per 1 part by weight of the component (C) is 0.05 to 1.5 parts by weight, preferably 0.09 to 1.5 parts by weight, more preferably 0.15 to 1.5 parts by weight, further preferably 0.19 to 1.5 parts by weight from the viewpoint of further improving the handleability of the agent when used and/or the crack suppression property during storage, or, in addition to this viewpoint, from the viewpoint of improving lathering and/or smoothness in rinsing of the solid hair detergent composition. The content of the component (B) per 1 part by weight of the component (C) may be, for example 0.05 to 0.71 parts by weight, preferably 0.05 to 0.51 parts by weight, more preferably 0.05 to 0.4 parts by weight, further preferably 0.05 to 0.35 parts by weight from the viewpoint of improving the moldability and/or lathering property of the solid hair detergent composition.

### (C) Water

The solid hair detergent composition of the present invention contains water as the component (C). The presence of water may cause the occurrence of cracks during storage. However, the solid hair detergent composition of the present invention is excellent in crack suppression property during storage.

In the solid hair detergent composition of the present invention, the content of the component (C) is not particularly limited as long as the hair detergent composition can be formulated in a solid form. For example, the content may be 5 to 25 wt%, preferably 7 to 23 wt%, more preferably 9 to 21.5 wt%, and further preferably 12 to 20 wt%, 14 to 19 wt%, or 16 to 18 wt%.

In the solid hair detergent composition of the present invention, the ratio between the component (A) and the component (C) is determined by the content of each component described above. The content of the component (C) per 100 parts by weight of the component (A) may be, for example 10 to 38 parts by weight, preferably 15 to 35 parts by weight, and more preferably 20 to 34 parts by weight, 22 to 32 parts by weight, or 24 to 30 parts by weight.

### (D) Cationized Polymer Other than Cationized Guar Gum

The solid hair detergent composition of the present invention may contain a cationized polymer other than cationized guar gum as the component (D). The cationized polymer other than cationized guar gum cannot improve the handleability of the agent when used by itself, but can further improve the handleability of the agent when used by being blended in the solid hair detergent composition that contains the components (A) and (C) together with the component (B). In addition, further blending the cationized polymer other than cationized guar gum can further improve the smoothness in rinsing.

The cationized polymer other than cationized guar gum is not particularly limited. Examples thereof include a polymer containing diallyl dimethyl quaternary ammonium halide as a constitutional unit, and cationized cellulose.

The molecular weight of the cationized polymer other than cationized guar gum is not particularly limited. Examples thereof include a weight average molecular weight of 1,000 to 1,000,000. From the viewpoint of further enhancing the effect of improving the handleability of the agent when used and/or further improving the smoothness in rinsing, the weight average molecular weight of the cationic polymer other than cationized guar gum may be preferably 10,000 to 500,000, more preferably 100,000 to 400,000, further preferably 150,000 to 250,000, and still further preferably 170,000 to 200,000. The weight average molecular weight is measured by gel permeation chromatography (GPC).

The degree of cationization of the cationized polymer other than cationized guar gum is not particularly limited. For example, the degree may be 4 meq/g or more in terms of cation density. From the viewpoint of further enhancing the effect of improving the handleability of the agent when used and/or further improving the smoothness in rinsing, the cation density of the cationized polymer other than cationized guar gum may be preferably 5 meq/g or more, more preferably 5.5 meq/g or more, and further preferably 6 meq/g or more. The upper limit of the range of the cation density of the cationized polymer other than cationized guar gum is not particularly limited. For example, the upper limit may be 10 meq/g or less, preferably 9 meq/g or less, more preferably 8 meq/g or less, further preferably 7 meq/g or less, still further preferably 6.5 meq/g or less, and particularly preferably 6.3 meq/g or less.

Among the above, the cationized polymer other than cationized guar gum is preferably a polymer containing a diallyl dimethyl quaternary ammonium halide as a constitutional unit from the viewpoint of further enhancing the effect of improving the handleability of the agent when used and/or further improving the smoothness in rinsing. Specific examples of the halide include chloride and bromide, and chloride is preferred.

Specific examples of the polymer containing a diallyldimethyl quaternary ammonium halide as a constitutional unit include dimethyldiallylammonium chloride homopolymer (polyquaternium-6), dimethyldiallylammonium chloride/acrylamide copolymer (polyquaternium-7), and dimethyldiallylammonium chloride/acrylic acid copolymer (polyquaternium-22). Among these polymers containing a diallyldimethyl quaternary ammonium halide as a constitutional unit, dimethyldiallylammonium chloride/acrylic acid copolymer (polyquaternium-22) is preferred from the viewpoint of further enhancing the effect of improving the handleability of the agent when used and/or further improving the smoothness in rinsing.

When the solid hair detergent composition of the present invention contains the component (D), the content of the component (D) is not particularly limited. For example, the content may be 0.1 to 5 wt%. From the viewpoint of further enhancing the effect of improving the handleability of the agent when used and/or further improving the smoothness in rinsing, the content of the component (D) may be preferably 0.15 to 4.5 wt%, more preferably 0.4 to 4.2 wt%, further preferably 0.6 to 4 wt%, still further preferably 1 to 4 wt% or 2 to 3 wt%.

When the solid hair detergent composition of the present invention contains the component (D), the content ratio between the component (A) and the component (D) is determined by the content of each component described above. From the viewpoint of further enhancing the effect of improving the handleability of the agent when used and/or further improving the smoothness in rinsing, the content of the component (D) per 100 parts by weight of the component (A) may be, for example 0.1 to 10 parts by weight, preferably 0.3 to 10 parts by weight, more preferably 0.9 to 10 parts by weight, further preferably 1 to 10 parts by weight, still further preferably 2 to 8 parts by weight, 3 to 6 parts by weight, 4 to 5 parts by weight, or 4.5 to 5 parts by weight.

### (E) Polyhydric Alcohol

The solid hair detergent composition of the present invention may contain a polyhydric alcohol as the component (E). The polyhydric alcohol can further improve the crack suppression property during storage of the solid hair detergent composition.

The polyhydric alcohol is not particularly limited. Examples thereof include dihydric alcohols and trihydric alcohols. Among these polyhydric alcohols, trihydric alcohols are preferred from the viewpoint of further enhancing the crack suppression property improving effect during storage and/or obtaining excellent smoothness in rinsing. Among these polyhydric alcohols, dihydric alcohols are preferred from the viewpoint of obtaining excellent moldability.

Dihydric alcohols are not particularly limited. Examples thereof include ethylene glycol, propylene glycol, and 1,3-butylene glycol. Among these dihydric alcohols, 1,3-butylene glycol is preferred from the viewpoint of obtaining excellent handleability when used. Trihydric alcohols are not particularly limited, and glycerin is preferred.

When the solid hair detergent composition of the present invention contains the component (E), the content of the component (E) is not particularly limited. For example, the content may be 0.1 to 15 wt%. From the viewpoint of further enhancing the effect of improving the crack suppression property during storage, the content of the (E) component may be preferably 0.5 to 15 wt%, more preferably 2.4 to 15 wt%, further preferably 5 to 15 wt%. From the viewpoint of obtaining excellent moldability, the content of the component (E) may be preferably 0.1 to 10 wt%, preferably 0.1 to 8 wt%, more preferably 0.1 to 7 wt%.

When the solid hair detergent composition of the present invention contains the component (E), the ratio between the component (A) and the component (E) is determined by the content of each component described above. The content of the component (E) per 100 parts by weight of the component (A) may be, for example 0.5 to 10 parts by weight, preferably 2 to 10 parts by weight, 4 to 10 parts by weight, more preferably 5 to 10 parts by weight, and further preferably 6 to 10 parts by weight from the viewpoint of further improving the crack suppression property during storage, and for example 0.5 to 7 parts by weight or 0.5 to 6 parts by weight from the viewpoint of obtaining excellent moldability.

When the solid hair detergent composition of the present invention contains the component (E), the content ratio between the component (C) and the component (E) is determined by the content of each component described above. The content of the component (E) per 1 part by weight of the component (C) may be, for example 0.05 to 0.5 parts by weight, preferably 0.1 to 0.5 parts by weight, more preferably 0.13 to 0.5 parts by weight, further preferably 0.3 to 0.5 parts by weight, still further preferably 0.39 to 0.5 parts by weight from the viewpoint of further enhancing the effect of improving the crack suppression property during storage, and preferably 0.05 to 0.42 parts by weight, more preferably 0.05 to 0.3 parts by weight, further preferably 0.05 to 0.22 parts by weight from the viewpoint of obtaining excellent moldability.

### (F) Coconut Oil Fatty Acid and/or (G) Alkali Metal Isethionate Salt

The solid hair detergent composition of the present invention may contain at least one of a coconut oil fatty acid as the component (F) and an alkali metal isethionate salt (examples thereof include sodium salts and potassium salts, and sodium salts are preferred) as the component (G).

When the solid hair detergent composition of the present invention contains the component (F), the content of the component (F) may be, for example 5 to 15 wt% and preferably 8 to 10 wt%.

When the solid hair detergent composition of the present invention contains the component (F), the ratio between the component (A) and the component (F) is determined by the content of each component described above. The content of the component (F) per 100 parts by weight of the component (A) may be, for example 10 to 20 parts by weight and preferably 13 to 16 parts by weight.

When the solid hair detergent composition of the present invention contains the component (G), the content of the component (G) may be, for example 0.5 to 2.5 wt% and preferably 1 to 2 wt%.

When the solid hair detergent composition of the present invention contains the component (G), the ratio between the component (A) and the component (G) is determined by the content of each component described above. The content of the component (G) per 100 parts by weight of the component (A) may be, for example 1 to 4 parts by weight and preferably 2 to 3.2 parts by weight.

### Other Components

The solid hair detergent composition of the present invention may contain, in addition to the above-described components (A) to (C) and the components (D) to (G) to be blended as necessary, an additional component normally used in the technical field of hair detergent compositions and/or solid detergents as long as the effects of the present invention are not impaired. Examples of such an additional component include surfactants (anionic surfactants other than the component (A), nonionic surfactants, amphoteric surfactants, etc.), oily bases (higher alcohols, ester oils, vegetable oils, hydrocarbon oils, etc.), higher fatty acids other than the component (F), thickeners other than the components (B) and (D), organic solvents, whitening agents, cell activators, anti-inflammatory agents, antibacterial agents, moisturizing agents, plant extracts, refreshing agents, inorganic powders, pearl agents, chelating agents, coloring agents, pigments, fragrances, deodorants, and antioxidants.

### Application

The solid hair detergent composition of the present invention is used for the purpose of cleansing hair or hair and scalp. When the solid hair detergent composition is used, an appropriate amount of the agent from the composition can be attached to hair or hair and scalp to wash the hair or the hair and scalp. Examples of the method for taking an appropriate amount of the agent include a method in which the solid hair detergent composition is rubbed against a palm together with an appropriate amount of water to take the agent, and a method in which the solid hair detergent composition of the present invention is directly rubbed against hair. Among these methods, a method in which the solid hair detergent composition of the present invention is rubbed against a palm is preferred because the solid hair detergent composition of the present invention is excellent in the handleability of the agent.

### Production Method

The solid hair detergent composition of the present invention is produced by mixing the above-described components (A) to (C), the components (D) to (G) to be blended as necessary, and other components to be blended as necessary, and preparing the mixture in a solid form.

### EXAMPLES

Hereinafter, the present invention will be more specifically described with reference to Examples. The present invention is not limited to these Examples.

Solid hair detergent compositions (30.0 g/piece, 5.8 cm × 4.2 cm × 1.0 cm) shown in Table 1 were prepared. Specifically, the components shown in Table 1 were pulverized and mixed by a three roll mill, then kneaded by an extruder set at 50°C, and molded by a pressing machine using a mold. The molded product was removed from the mold and naturally cooled to room temperature to produce a solid hair detergent composition. The details of the ingredients used in each solid hair detergent composition are as follows.
- Sodium cocoyl isethionate: component in PUREACT I-78 (Innospec Active Chemicals LLC)
- Sodium lauroyl glutamate: Aminosurfact ALMS-P1 (Asahi Kasei Finechem Co., Ltd.)
- Sodium lauroyl aspartate: AminoFoamer FLMS-P1 (Asahi Kasei Finechem Co., Ltd.)
- Guar hydroxypropyltrimonium chloride: CATINAL CG-100LD (TOHO Chemical Industry Co., Ltd.)
- Polyquaternium-10: POIZ C-150L (Kao Corporation)
- Polyquaternium-22: Merquat 295 (Lubrizol Japan Limited), cation density 6.0 meq/g, weight average molecular weight 190,000
- Coconut oil fatty acid: component in PUREACT I-78
- Sodium Isethionate: component in PUREACT I-78

The obtained solid hair detergent compositions were evaluated as follows for the handleability when used, crack suppression property during storage, moldability, lathering, and smoothness in rinsing. The results are shown in Table 1.

### <Handleability of agent when used>

Each solid hair detergent composition was thoroughly wetted with 100 g of water, and after cutting off water droplets, the solid hair detergent composition was rubbed back and forth for 5 times on a palm, and the amount of the agent transferred to the hand was evaluated on a scale of 1 to 10, in which 10 points were given to the amount of the agent in the case of the solid hair detergent composition of Example 4, and 1 point was given to the amount of the agent in the case of the solid hair detergent composition of Comparative Example 1. It is determined that the higher the score is, the more excellent the handleability of the agent when used is. When the score was 6 points or more, it was determined that the desired handleability was achieved.

### <Crack Suppression Property During Storage>

Each solid hair detergent composition was immersed in water having a temperature of 5°C for 2 hours, then taken out from the water, and allowed to stand in an environment having a temperature of 5°C and a humidity of 30 %RH for 1 day. Thereafter, the crack density (ratio of the area of cracks to the surface area of the solid hair detergent composition) in the solid hair detergent composition was evaluated on a scale of 1 to 10, in which 10 points were given to the case where there was no crack, and 1 point was given to the case where the composition had cracks to the degree of the crack density of the solid hair detergent composition of Comparative Example 2 (many large cracks occurred). It is determined that the larger the score is, the better the crack suppression property during storage is. When the score was 5 points or more, it was determined that there was no problem as an actual product with respect to the crack suppression property during storage.

### <Moldability>

The difference between the shape of each solid hair detergent composition and the shape of the mold and the degree of the difference were evaluated. Specifically, the degree of divergence from the mold shape was evaluated on a scale of 1 to 5, in which 5 points were given to the case where the shape of the solid hair detergent composition was molded according to the shape of the mold and there was no divergence from the mold shape (neither indentation nor chipping was observed), and 1 point was given to the case where the composition had a divergence from the mold shape due to indentation and/or chipping to the degree of the divergence found in the solid hair detergent composition of Comparative Example 1. It is determined that the larger the score is, the more excellent the moldability is, and the more excellent the beauty of the appearance of the solid hair detergent composition is.

### <Lathering>

A lathering operation was performed on 0.5 g of each solid hair detergent composition in the palm of a hand for 10 seconds using 100 g of water. The amount of the lather produced by the lathering operation was evaluated on a scale of 1 to 10, in which 10 points were given to the amount of the lather produced by the solid hair detergent composition of Example 13, and 1 point was given to the case where lather was not produced. It is determined that the higher the score is, the more excellent in lathering the solid hair detergent composition is.

### <Smoothness in Rinsing>

Hair was washed with 1.0 g of each solid hair detergent composition and rinsed with hot water (38°C). Smoothness of the hair in rinsing was evaluated on a scale of 1 to 10, in which 10 points (very smooth) were given to the solid hair detergent composition of Example 4, and 1 point (coarse) was given to the solid hair detergent composition of Comparative Example 2. It is determined that the higher the score is, the better the smoothness of the solid hair detergent composition in rinsing is.

**[Table 1]**

| | | Examples | | | | | | | | | | | | | |
|---|---|---|---|---|---|---|---|---|---|---|---|---|---|---|---|
| | | 1 | 2 | 3 | 4 | 5 | 6 | 7 | 8 | 2 | 9 | 10 | 11 | 12 | 13 |
| (A) | Sodium cocoyl isethionate | 100 | 100 | 100 | 100 | 100 | 100 | 100 | 100 | 100 | 100 | 100 | 100 | 100 | 100 |
| | | (58.7) | (67.2) | (63.1) | (61.2) | (63.1) | (63.1) | (69.5) | (68.3) | (67.2) | (65.1) | (63.1) | (72.5) | (72.1) | (72.0) |
| | Sodium lauroyl glutamate | - | - | - | - | - | - | - | - | - | - | - | - | - | - |
| | Sodium lauroyl aspartate | - | - | - | - | - | - | - | - | - | - | - | - | - | - |
| (B) | Guar hydroxypropyltrimonium chloride | 6.7 | 7.3 | 7.3 | 7.3 | 7.3 | 7.3 | 2.4 | 4.9 | 7.3 | 12.2 | 17.1 | 8.9 | 8.9 | 8.9 |
| (D) | Polyquaternium-10 | - | - | - | - | - | - | - | - | - | - | - | - | - | - |
| (D) | Polyquaternium-22 | 6.7 | - | - | 4.9 | - | - | - | - | - | - | - | - | - | 0.3 |
| (E) | Glycerin | 6.7 | - | 9.8 | 9.8 | - | - | - | - | - | - | - | - | 0.6 | 0.6 |
| (E) | Propylene glycol | - | - | - | - | 9.8 | - | - | - | - | - | - | - | - | - |
| (E) | 1,3-butylene glycol | - | - | - | - | - | 9.8 | - | - | - | - | - | - | - | - |
| (F) | Coconut oil fatty acid | 14.7 | 14.6 | 14.6 | 14.6 | 14.6 | 14.6 | 14.6 | 14.6 | 14.6 | 14.6 | 14.6 | 13.9 | 13.9 | 13.9 |
| (G) | Sodium isethionate | 2.3 | 2.4 | 2.4 | 2.4 | 2.4 | 2.4 | 2.4 | 2.4 | 2.4 | 2.4 | 2.4 | 2.5 | 2.5 | 2.5 |
| (C) | Water | 33.3 | 24.4 | 24.4 | 24.4 | 24.4 | 24.4 | 24.4 | 24.4 | 24.4 | 24.4 | 24.4 | 12.7 | 12.7 | 12.7 |
| | | (19.6) | (16.4) | (15.4) | (14.9) | (15.4) | (15.4) | (16.9) | (16.7) | (16.4) | (15.9) | (15.4) | (9.2) | (9.1) | (9.1) |
| Total | | 170 | 149 | 158 | 163 | 159 | 159 | 144 | 146 | 149 | 154 | 159 | 138 | 139 | 139 |
| (B) part by weight / (C) 1 part by weight | | 0.20 | 1.30 | 0.30 | 0.30 | 0.30 | 0.30 | 0.10 | 0.20 | 0.30 | 0.50 | 0.70 | 0.70 | 0.70 | 0.70 |
| (E) part by weight / (C) 1 part by weight | | 0.20 | - | 0.40 | 0.40 | 0.40 | 0.40 | - | - | - | - | - | - | 0.05 | 0.05 |
| Handleability when used (scale of 1 to 10) | | 10 | 8 | 8 | 10 | 6 | 8 | 6 | 6 | 8 | 8 | 8 | 8 | 8 | 10 |
| Crack suppression property during storage (scale of 1 to 10) | | 10 | 8 | 10 | 10 | 9 | 9 | 7 | 8 | 8 | 8 | 8 | 5 | 6 | 6 |
| Moldability (scale of 1 to 5) | | 4 | 4 | 2 | 2 | 4 | 4 | 4 | 5 | 4 | 2 | 2 | 4 | 4 | 5 |
| Lathering (scale of 1 to 10) | | 9 | 10 | 9 | 9 | 9 | 9 | 8 | 8 | 10 | 9 | 8 | 9 | 10 | 10 |
| Smoothness in rinsing (scale of 1 to 10) | | 10 | 10 | 9 | 10 | 7 | 7 | 7 | 8 | 10 | 9 | 10 | 7 | 7 | 9 |

In the table, the unit of numerical value showing the blending amount of each component is "part by weight" unless otherwise noted, and the unit of numerical value in brackets is "wt%".

| | | Comparative Examples | | | | |
|---|---|---|---|---|---|---|
| | | 1 | 2 | 3 | 4 | 5 |
| (A) | Sodium cocoyl isethionate | - | - | 100 | 100 | 100 |
| | | | | (58.7) | (67.2) | (67.2) |
| | Sodium lauroyl glutamate | 100 | - | - | - | - |
| | | (75.9) | | | | |
| | Sodium lauroyl aspartate | - | 100 | | | |
| | | | (75.9) | | | |
| (B) | Guar hydroxypropyltrimonium chloride | 7.3 | 7.3 | - | - | |
| (D) | Polyquaternium-10 | - | - | 6.7 | 7.3 | |
| (D) | Polyquaternium-22 | - | - | 6.7 | - | 7.3 |
| (E) | Glycerin | - | - | 6.7 | - | - |
| (E) | Propylene glycol | - | - | - | - | |
| (E) | 1,3-butylene glycol | - | - | - | - | |
| (F) | Coconut oil fatty acid | - | - | 14.7 | 14.6 | 14.6 |
| (G) | Sodium isethionate | - | - | 2.3 | 2.4 | 2.4 |
| (C) | Water | 24.4 | 24.4 | 33.3 | 24.4 | 24.4 |
| | | (18.5) | (18.5) | (19.6) | (16.4) | (16.4) |
| Total | | 132 | 132 | 170 | 149 | 149 |
| (B) part by weight / (C) 1 part by weight | | 0.30 | 0.30 | - | - | - |
| (E) part by weight / (C) 1 part by weight | | - | - | 0.20 | - | - |
| Handleability when used (scale of 1 to 10) | | 1 | 6 | | 6 | 4 |
| Crack suppression property during storage (scale of 1 to 10) | | 7 | 1 | 8 | 3 | 8 |
| Moldability (scale of 1 to 5) | | 1 | 2 | 5 | 4 | 3 |
| Lathering (scale of 1 to 10) | | 3 | 4 | 8 | 6 | 6 |
| Smoothness in rinsing (scale of 1 to 10) | | 2 | 1 | 2 | 2 | 1 |

In the table, the unit of numerical value showing the blending amount of each component is "part by weight" unless otherwise noted, and the unit of numerical value in brackets is "wt%".

## Claims

1. A solid hair detergent composition comprising (A) an alkali metal cocoyl isethionate salt, (B) cationized guar gum, and (C) water,
wherein the content of the component (A) is 55 to 80 wt%, and
wherein the content of the component (B) is 0.05 to 1.5 parts by weight per 1 part by weight of the component (C).

2. The solid hair detergent composition according to claim 1, wherein a content of the component (B) is 1 to 20 parts by weight per 100 parts by weight of the component (A).

3. The solid hair detergent composition according to claim 1 or 2, the composition comprising 5 to 25 wt% of the component (C).

4. The solid hair detergent composition according to any one of claims 1 to 3, further comprising (D) a cationized polymer other than cationized guar gum.

5. The solid hair detergent composition according to claim 4, wherein the component (D) is a polymer containing a diallyl dimethyl quaternary ammonium halide as a constitutional unit.

6. The solid hair detergent composition according to claim 4 or 5, wherein a content of the component (D) is 0.1 to 10 parts by weight per 100 parts by weight of the component (A).

7. The solid hair detergent composition according to any one of claims 1 to 6, further comprising (E) a polyhydric alcohol.

8. The solid hair detergent composition according to claim 7, wherein a content of the component (E) is 0.5 to 10 parts by weight per 100 parts by weight of the component (A).

9. The solid hair detergent composition according to claim 7 or 8, wherein the content of the component (E) is 0.05 to 0.5 parts by weight per 1 part by weight of the component (C).

10. The solid hair detergent composition according to any one of claims 1 to 9, further comprising (F) a coconut oil fatty acid and/or (G) an alkali metal isethionate salt.

## Patentansprüche

1. Feste Haarwaschmittelzusammensetzung, umfassend (A) ein Alkalimetall-Cocoylisethionatsalz, (B) kationisiertes Guarkernmehl und (C) Wasser,
wobei der Anteil der Komponente (A) 55 bis 80 Gew.- % beträgt, und
wobei der Anteil der Komponente (B) 0,05 bis 1,5 Gewichtsteile pro 1 Gewichtsteil der Komponente (C) beträgt.

2. Feste Haarwaschmittelzusammensetzung nach Anspruch 1, wobei der Anteil der Komponente (B) 1 bis 20 Gewichtsteile pro 100 Gewichtsteile der Komponente (A) beträgt.

3. Feste Haarwaschmittelzusammensetzung nach Anspruch 1 oder 2, wobei die Zusammensetzung 5 bis 25 Gew.- % der Komponente (C) enthält.

4. Feste Haarwaschmittelzusammensetzung nach einem der Ansprüche 1 bis 3, weiter umfassend (D) ein kationisiertes Polymer, das kein kationisiertes Guarkernmehl ist.

5. Feste Haarwaschmittelzusammensetzung nach Anspruch 4, wobei die Komponente (D) ein Polymer ist, das ein Diallyldimethyl-quaternäres Ammoniumhalogenid als eine konstitutive Einheit enthält.

6. Feste Haarwaschmittelzusammensetzung nach Anspruch 4 oder 5, wobei der Anteil der Komponente (D) 0,1 bis 10 Gewichtsteile pro 100 Gewichtsteile der Komponente (A) beträgt.

7. Feste Haarwaschmittelzusammensetzung nach einem der Ansprüche 1 bis 6, weiter umfassend (E) einen mehrwertigen Alkohol.

8. Feste Haarwaschmittelzusammensetzung nach Anspruch 7, wobei der Anteil der Komponente (E) 0,5 bis 10 Gewichtsteile pro 100 Gewichtsteile der Komponente (A) beträgt.

9. Feste Haarwaschmittelzusammensetzung nach Anspruch 7 oder 8, wobei der Anteil der Komponente (E) 0,05 bis 0,5 Gewichtsteile pro 1 Gewichtsteil der Komponente (C) beträgt.

10. Feste Haarwaschmittelzusammensetzung nach einem der Ansprüche 1 bis 9, weiter umfassend (F) eine Kokosnussölfettsäure und/oder (G) ein Alkalimetallisethionatsalz.

## Revendications

1. Composition de détergent capillaire solide comprenant (A) un sel d'iséthionate de cocoyle de métal alcalin, (B) de la gomme de guar cationisée et (C) de l'eau, dans laquelle la teneur en le composant (A) est de 55 à 80 % en poids, et
dans laquelle la teneur en le composant (B) est de 0,05 à 1,5 partie en poids pour 1 partie en poids du composant (C).

2. Composition de détergent capillaire solide selon la revendication 1, dans laquelle une teneur en le composant (B) est de 1 à 20 parties en poids pour 100 parties en poids du composant (A).

3. Composition de détergent capillaire solide selon la revendication 1 ou 2, la composition comprenant 5 à 25 % en poids du composant (C).

4. Composition de détergent capillaire solide selon l'une quelconque des revendications 1 à 3, comprenant en outre (D) un polymère cationisé autre que de la gomme de guar cationisée.

5. Composition de détergent capillaire solide selon la revendication 4, dans laquelle le composant (D) est un polymère contenant un halogénure d'ammonium quaternaire de diallyldiméthyle en tant qu'unité constitutive.

6. Composition de détergent capillaire solide selon la revendication 4 ou 5, dans laquelle une teneur en le composant (D) est de 0,1 à 10 parties en poids pour 100 parties en poids du composant (A).

7. Composition de détergent capillaire solide selon l'une quelconque des revendications 1 à 6, comprenant en outre (E) un alcool polyhydrique.

8. Composition de détergent capillaire solide selon la revendication 7, dans laquelle une teneur en le composant (E) est de 0,5 à 10 parties en poids pour 100 parties en poids du composant (A).

9. Composition de détergent capillaire solide selon la revendication 7 ou 8, dans laquelle la teneur en le composant (E) est de 0,05 à 0,5 partie en poids pour 1 partie en poids du composant (C).

10. Composition de détergent capillaire solide selon l'une quelconque des revendications 1 à 9, comprenant en outre (F) un acide gras d'huile de coco et/ou (G) un sel d'iséthionate de métal alcalin.
